(19) Europäisches Patentamt
European Patent Office
Office européen des brevets

(11) Publication number: **0 482 605 A2**

(12) **EUROPEAN PATENT APPLICATION**

(21) Application number: **91118065.1**

(22) Date of filing: **23.10.91**

(51) Int. Cl.[5]: **C07K 7/08**, C07K 7/10, G01N 33/569

(30) Priority: **24.10.90 JP 287836/90**

(43) Date of publication of application: **29.04.92 Bulletin 92/18**

(84) Designated Contracting States: **CH DE FR GB IT LI NL**

(71) Applicant: **Kuraray Co., Ltd.**
**1621, Sakazu**
**Kurashiki-shi Okayama-ken(JP)**

(72) Inventor: **Maeda, Yoshiaki**
**3-39-13, Hiigawa, Jonan-ku**
**Fukuoka-shi, Fukuoka-ken(JP)**
Inventor: **Shiraki, Hiroshi**
**2-20-22, Tsutsujigaoka**
**Ononjo-shi, Fukuoka-ken(JP)**
Inventor: **Inoue, Yukiko**
**705 467-1, Musashi**
**Chikushino-shi, Fukuoka-ken(JP)**
Inventor: **Kuroda, Naotaka**
**Shimooridanchi 6-505**
**Onojo-shi, Fukuoka-ken(JP)**
Inventor: **Kiyokawa, Hiroyuki**
**37-13, 4-chome, Arita, Sawara-ku**
**Fukuoka-shi, Fukuoka-ken(JP)**
Inventor: **Matsumoto, Koji**
**Shimooridanchi 43-104**
**Onojo-shi, Fukuoka-ken(JP)**

(74) Representative: **Vossius & Partner**
**Siebertstrasse 4 P.O. Box 86 07 67**
**W-8000 München 86(DE)**

(54) **HTLV-II derived peptide.**

(57) The present invention is directed to a peptide comprising a HTLV-II-derived peptide fragment capable of specifically binding to anti-HTLV-II antibody, which does not react with anti-HTLV-I antibody.

The peptide of the present invention can be used as an assay reagent for the detection of anti-HTLV-II antibody.

EP 0 482 605 A2

The present invention relates to a peptide and its use.

The peptide provided by the present invention is a novel peptide capable of specifically binding only to the antibody having specificity to antigens associated with human T cell leukemia virus type II (hereinafter referred to as HTLV-II), which antibody is hereinafter referred to as anti-HTLV-II antibody. The peptide of the present invention can be used for specific determination of anti-HTLV-II antibody, since it does not show cross-reaction with the antibody having specificity to antigens associated with human T cell leukemia virus type I (hereinafter referred to as HTLV-I), which antibody is hereinafter referred to as anti-HTLV-I antibody.

The assay reagent for anti-HTLV-II antibody provided by the present invention is capable of detecting anti-HTLV-II antibody in serum or plasma with high sensitivity and is thus useful in the determination of anti-HTLV-II antibody.

HTLV-I and HTLV-II are associated with specific forms of human malignant tumor. HTLV-I is the etiologic agent for a malignant tumor known as adult T cell leukemia and for a chronic myelopathy known as HTLV-I-associated myelopathy or tropical spastic paralysis [Proceedings of the National Academy of Science of the United States of America, 78, 6476 (1981); Lancet, 7, 104-105 (1986); Lancet, 2, 407-410 (1985)]. On the other hand, HTLV-II as pathogen has been reported only in rare patients having an unusual T cell malignancy known as hairy-cell leukemia [Science, 218, 571-573 (1982)]. However, recent epidemiological survey using the polymerase chain reaction **for nucleic acid** amplification showed that the majority of drug abusers with HTLV-reactive antibodies are infected with HTLV-II rather than HTLV-I [Science, 244, 471-475 (1989)]. This study suggests that some of the regions where HTLV-I infection is endemic may be regions where HTLV-II infection is also endemic. Simple serologic tests that clearly distinguish among these infectious diseases are essential for clarifying their transmission patterns and classifying them for pathogenesis patterns. However, the major limitation to serological diagnosis of HTLV-II has been the difficulty in distinguishing HTLV-II infection from HTLV-I infection, because of substantial cross-reactivity owing to the relatively high amino acid sequence homology between the two viruses [Science, 225, 571-573 (1982)]. In the conventional serological diagnostic methods based on an antigen-antibody reaction using whole virus lysates as antigens, it is impossible to clearly discriminate HTLV-II infection from HTLV-I infection, due to partial cross-reactivity against antigens having a well-conserved amino acid sequence. Accordingly, HTLV-I is closely associated with HTLV-II, and protein coding regions of HTLV-II provirus show about 60% homology with those of HTLV-I at the nucleotide level [Proceedings of the National Academy of Science of the United States of America, 82, 3101-3105 (1985)].

The gag precursor protein of HTLV-II is cleaved to 15-, 24- and 9- kDa proteins, which show 55%, 85% and 68% homology, respectively, with the corresponding protein of HTLV-I. Also, the amino acid sequence homology of the surface glycoproteins and membrane proteins predicted from proteolytic sites of the env precursor proteins of the two retroviruses are 63% and 73%, respectively. These facts suggest that the assay using whole virus lysates as antigens will not reliably distinguish between HTLV-I infection and HTLV-II infection.

Synthetic peptide has some potential advantages over authentic viral proteins. Because **the preparation of synthetic peptides** is not based on biological techniques, it contains less amounts of contaminants which can cause non-specific antibody adsorption. Thus, the present inventors selected a site showing high antigenicity in the peptide of HTLV-II and attempted to synthesize a peptide corresponding to this site.

It is an object of the present invention to provide a peptide capable of specifically binding to anti-HTLV-II antibody. It is another object of the invention to provide an assay reagent for anti-HTLV-II antibody.

According to the present invention, the objects described above can be accomplished by providing a peptide comprising a HTLV-II-derived peptide fragment which does not react with anti-HTLV-I antibody but which is capable of specifically binding to anti-HTLV-II antibody, for example:

1) the peptide represented by the formula:

Phe Ile Thr Ser Glu Pro Thr Gln Pro Pro Pro Thr Ser Pro Pro Leu Val His Asp Ser Asp Leu Glu His Val Leu (SEQ ID NO:1),

2) the peptide represented by the formula:

Pro Pro Ser Pro Glu Ala His Val Pro Pro Pro Tyr (SEQ ID NO:2), and

3) an assay reagent for anti-HTLV-II antibody comprising one of the above peptides.

Each abbreviation of amino acid residues used in this specification means the following respectively:

| | | | |
|---|---|---|---|
| Ala | : L-alanine residue | Arg | : L-arginine residue |
| Asn | : L-asparagine residue | Asp | : L-aspartic acid residue |
| Cys | : L-cysteine residue | Gln | : L-glutamine residue |
| Glu | : L-glutamic acid residue | Gly | : glycine residue |
| His | : L-histidine residue | Ile | : L-isoleucine residue |
| Leu | : L-leucine residue | Lys | : L-lysine residue |
| Met | : L-methionine residue | Phe | : L-phenylalanine residue |
| Pro | : L-proline residue | Ser | : L-serine residue |
| Thr | : L-threonine residue | Trp | : L-tryptophan residue |
| Tyr | : L-tyrosine residue | Val | : L-valine residue |

In the present specification, amino acid sequences are described so that the N-terminal amino acid residue is located on the left and the C-terminal amino acid residue is located on the right in accordance with the common practice.

Of the peptides of the present invention, the peptide represented by SEQ ID NO:1 and the peptide represented by SEQ ID NO:2 correspond to the peptide fragments encoded by the env gene and gag gene of HTLV-II virus, respectively. The peptides of the present invention are capable of specifically binding to anti-HTLV-II antibody alone. As long as such binding capability is possessed, the peptides of the present invention include those in which amino acid substitution, insertion or deletion exists in one or more of the amino acids constituting them. Here, the capability of specifically binding to anti-HTLV-II antibody alone means the nature that the peptide binds to anti-HTLV-II antibody and does not bind at least to anti-HTLV-I antibody.

The peptide of the present invention can be synthesized by an ordinary method of peptide synthesis such as the solid phase synthesis method or a liquid phase synthesis method such as the stepwise elongation method or the fragment condensation method, but the solid phase synthesis method is preferred since it is simple to operate [Journal of the American Chemical Society, vol. 85, pp. 2149-2154 (1963); Seikagaku Jikken Koza 1: Tanpakushitsu no Kagaku IV, Kagaku Shusyoku to Pepuchido Gosei, edited by the Japanese Biochemical Society, published by Tokyo Kagaku Dojin, November 15, 1977, pp. 207-495; Zoku Seikagaku Jikken Koza 2: Tanpakushitsu no Kagaku (II), edited by the Japanese Biochemical Society, published by Tokyo Kagaku Dojin, May 20, 1987, pp. 641-694].

Production of the peptide of the present invention by the solid phase synthesis method is carried out, for example, by repeating the process of binding an amino acid corresponding to the C-terminal of the desired peptide or the amide thereof to a polymer insoluble in reaction solvent such as styrene-divinylbenzene copolymer via the α-COO-group or α-CONH- group obtained by eliminating the hydrogen atom from the α-COOH group or α-$CONH_2$ group contained therein and subsequently condensing and binding the corresponding amino acid or peptide fragment to the amino acid or its amide in the direction of the N-terminal of the desired peptide after protecting the functional group other than the α-COOH group contained in the amino acid or peptide fragment such as α-amino acid and the process of eliminating the protective group bound to the amino group which forms the peptide linkage, such as α-amino group, in the

bound amino acid or peptide fragment to elongate the peptide chain to a peptide chain corresponding to the desired peptide, then eliminating the peptide chain from the polymer and removing the protective group from the protected functional group to yield the desired peptide, which is then purified. Here, it is preferable from the viewpoint of suppression of side reaction that the elimination of the peptide chain from the polymer and the removal of the protective group be conducted simultaneously using hydrogen fluoride. Also, it is efficient to purify the obtained peptide by reversed phase liquid chromatography.

Because the peptides of the present invention do not react with anti-HTLV-I antibody and are capable of specifically binding to anti-HTLV-II antibody, they permit specific detection of only HTLV-II carriers and can be used as an assay reagent to distinguish between HTLV-I infection and HTLV-II infection.

When using a peptide of the present invention as an assay reagent for anti-HTLV-II antibody, any of SEQ ID NO:1 and SEQ ID NO:2 can be used, but the peptide of SEQ ID NO:1 is preferably used, since it is more reactive with sera from HTLV-II carriers as seen in Examples given follow.

Determination of anti-HTLV-II antibody using a peptide of the present invention is performed by using either of the fluorescent immunoassay method (hereinafter referred to as the IF method), the gelatin particle agglutination method (hereinafter referred to as the PA method), the radioimmunoassay method and the enzyme-linked immunosorbent assay (hereinafter referred to as ELISA). All these methods are known. In the case of ELISA, it is preferable to use microwells or beads of glass or resin as the sorbent. The ELISA using microwells as the sorbent, for example, is described below.

The entire assay system comprises the microwells, the peptide of the present invention as the assay reagent, a blocking agent, a subject sample, a labeled antibody, an enzyme, and a coloring agent. The inner surface of the microwells is coated with the peptide of the present invention and then reacted with the blocking agent to block the nonspecific protein binding site on the surface. The subject sample is added to the peptide-coated microwells, followed by incubation. After removing the sample and washing the microwells, the enzyme-labeled antibody is added to the microwells, followed by incubation. After removing the reagent and washing the microwells, the coloring agent is then added to the microwells, followed by incubation. The amount of reaction product produced in the reaction between the enzyme and the coloring agent is determined using a spectrometer. Prior to assay runs, the peptide of the present invention is dissolved in a 0.01 M carbonate buffer. The resulting solution is added to, for example, a polystyrene microwell and then kept standing at 4°C overnight or at room temperature for 3 hours, whereby the surface of the microwell is coated with the peptide of the present invention. Examples of the blocking agent to block the nonspecific protein binding site include bovine serum albumin, casein, powdered skim milk, serum of the animals immunized to produce antibodies for enzyme labeling, and gelatin. Examples of the antibodies for enzyme labeling include antihuman IgG antibody and antihuman IgM antibody. Examples of the enzyme include alkaline phosphatase, glucose oxidase, peroxidase and beta galactosidase. It is preferable to prepare a conjugate as a part of the entire assay system by binding the enzyme to the labeling antibody using a compound having two or more functional groups, such as glutaraldehyde, before assay runs. An appropriate coloring agent is selected according to the selected enzyme. For example, when selecting peroxidase as the enzyme, o-phenylenediamine is preferred.

As stated above, the present invention provides a peptide capable of specifically binding to anti-HTLV-II antibody. This peptide makes it possible to provide an assay reagent for anti-HTLV-II antibody.

## EXAMPLES

The present invention is hereinafter described in more detail by means of the following examples, but these are not to be construed as limitative on the present invention.

### Example 1

The peptide of the present invention represented by the formula:

Phe Ile Thr Ser Glu Pro Thr Gln Pro Pro Pro Thr Ser Pro

Pro Leu Val His Asp Ser Asp Leu Glu His Val Leu (SEQ ID

NO:1),

was synthesized by the solid phase synthesis method using an automatic peptide synthesizer [model 431A, product of Applied Biosystems Inc., USA] as follows.

To 694 mg of a particulate resin (produced by Applied Biosystems, USA, PAM Leucine, t-Boc-L-Leu) comprising a styrene-divinyl benzene copolymer (styrene:divinyl benzene molar ratio = 99:1) containing 0.72 mmol/g resin of 4-[N-(t-butoxycarbonyl)-L-leucyloxymethyl]phenylacetamidomethyl group:

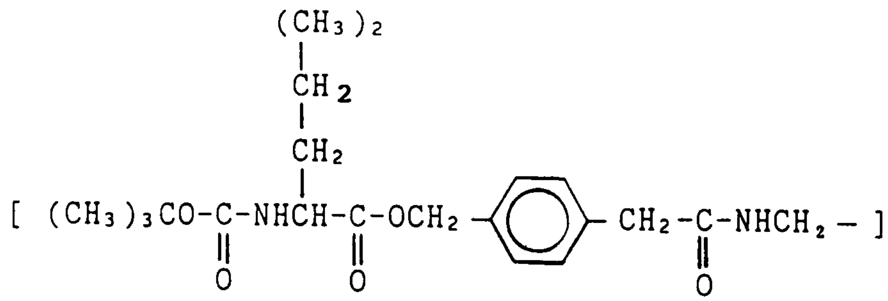

the corresponding amino acids, namely L-aspartic acid, L-glutamine, L-glutamic acid, L-histidine, L-isoleucine, L-leucine, L-phenylalanine, L-proline, L-serine, L-threonine and L-valine were bound in this order in the direction of the N-terminal of the desired peptide in accordance with the series of procedures shown in Table 1. In the condensation reaction, these amino acids were used as N-(t-butoxycarbonyl)-L-aspartic acid-$\beta$-benzyl ester,
N-$\alpha$(t-butoxycarbonyl)-L-glutamine,
N-(t-butoxycarbonyl)-L-glutamic acid-$\gamma$-benzyl ester,
N-$\alpha$(t-butoxycarbonyl)-N-im-dinitrophenyl-L-histidine.
N-(t-butoxycarbonyl)-L-isoleucine,
N-(t-butoxycarbonyl)-L-leucine,
N-(t-butoxycarbonyl)-L-phenylalanine,
N-(t-butoxycarbonyl)-L-proline,
N-(t-butoxycarbonyl)-O-benzyl-L-serine,
N-(t-butoxycarbonyl)-O-benzyl-L-threonine and
N-(t-butoxycarbonyl)-L-valine, respectively. The amount of their use was about 4 times that of the substrate on a molar basis. The condensation reaction was carried out at room temperature. Reaction time ranged from 100 to 110 minutes. After completion of the reaction procedures for all amino acids, the resulting resin was washed by sequential addition of diethyl ether, dichloromethane and methanol on glass filter, followed by vacuum drying to yield 2.05 g of a dry resin. In a glass flask, 1.0 g of this dry resin was mixed with 2 ml of thiophenol and 48 ml of N,N-dimethylformamide, followed by stirring at room temperature using a stirrer for 30 minutes. After supernatant removal, the same procedure was repeated three times. The residue was washed with dichloromethane and methanol, after which it was dried under reduced pressure to yield 1.0 g of a resin. Then, 1.0 g of the obtained dry resin was mixed with 1.5 ml of anisole and 0.25 ml of ethyl methyl sulfide in a polytrifluoromonochloroethylene reaction vessel (HF-reactor I type, product of Peptide Institute, Inc.). To this mixture 10 ml of hydrogen fluoride was added at a temperature of -20 °C, followed by stirring at that temperature for 30 minutes and then at 0 °C for 30 minutes. From the resulting reaction mixture, the hydrogen fluoride, anisole and ethyl methyl sulfide were evaporated off under reduced pressure, and the resulting residue was thoroughly washed with diethyl ether on a glass filter. The washed residue was extracted with 2 N aqueous acetic acid, and the extract was lyophilized to yield 780 mg of a crude peptide. The crude product thus obtained was subjected to preparative reversed phase high performance liquid chromatography [column: packed with $\mu$ BONDASPHERE 15$\mu$ C18-100A(Waters, octadecylated silica gel, 15$\mu$m in grain diameter), inside diameter 50 mm, length 300 mm; mobile phase: acetonitrile-water mixed solvent containing 0.05% by volume of trifluoroacetic acid (the acetonitrile density was gradually changed from 20% by volume to 40% by volume over a period of 30 minutes); flow rate: 5 ml/min; detection method: spectrophotometry at a wavelength of 210 nm], whereby 540 mg of a purified product of the desired peptide was obtained. The obtained purified product was subjected to analytical reversed phase high performance liquid chromatography [column: packed with TSK-gel ODS-80TM (Tosoh Corporation, octadecylated silica gel, 5$\mu$m in grain diameter), inside diameter 4 mm, length 150 mm; mobile phase: acetonitrile-water mixed solvent containing 0.05% by volume of trifluoroacetic acid (the acetonitrile density was gradually changed from 5% by volume to 50% by volume over a period of 30 minutes); flow

rate: 1 ml/min; detection method: spectrophotometry at a wavelength of 210 nm]; a single acute peak appeared at a retention time of 20.6 minutes. The molecular weight of the purified product was determined to be 2853 by fast atomic bombardment (hereinafter abbreviated FAB) mass spectrometry (theoretical value = 2853.03).

Table 1

| Process | Solvent and/or reagent used (%: by volume) | Time (min) | Frequency |
|---|---|---|---|
| 1: Removal of t-butoxycarbonyl group | Dichloromethane containing 50-65% of Trifluoroacetic acid | 16 | 1 |
| 2: Washing | Dichloromethane | 3 | 5 |
| 3: Neutralization | N-methylpyrrolidone containing 5% of diisopropylethylamine | 4 | 1 |
| 4: Washing | N-methylpyrrolidone | 5 | 6 |
| 5: Condensation | N-methylpyrrolidone containing hydroxybenzotriazole ester of (t-butoxycarbonyl)-amino acid | 39 | 1 |
| 6: Removal of unreacted amine | N-methylpyrrolidone containing 10% of acetic acid and 5% of diisopropylethylamine | 9 | 1 |
| 7: Washing | Dichloromethane | 4 | 6 |

Example 2

The peptide of the present invention represented by the formula:

Pro Pro Ser Pro Glu Ala His Val Pro Pro Pro Tyr (SEQ ID NO:2),

was obtained by solid phase synthesis and purification of peptide in the same manner as in Example 1. The obtained peptide was subjected to analytical reversed phase high performance liquid chromatography [conditions were the same as above]; a single acute peak appeared at a retention time of 17.1 minutes. The molecular weight of the peptide was determined to be 1286 by FAB mass spectrometry (theoretical value = 1286.39).

Reference Example 1

The HTLV-I-associated peptide represented by the formula:

Phe Leu Asn Thr Glu Pro Ser Gln Leu Pro Pro Thr Ala Pro Pro Leu Leu Pro His Ser Asn Leu Asp His Ile (SEQ ID NO:3),

was obtained by solid phase synthesis and purification of peptide in the same manner as in Example 1. The obtained peptide was subjected to analytical reversed phase high performance liquid chromatography [conditions were the same as above]; a single acute peak appeared at a retention time of 18.6 minutes. The molecular weight of the peptide was determined to be 2749 by FAB mass spectrometry (theoretical value = 2748.96).

Reference Example 2

The HTLV-I-associated peptide represented by the formula:

Pro Pro Pro Pro Ser Ser Pro Thr His Asp Pro Pro Asp Ser Asp Pro Gln Ile Pro Pro Pro Tyr Val Glu Pro Thr Ala Pro Gln Val Leu (SEQ ID NO:4),

was obtained by solid phase synthesis and purification of peptide in the same manner as in Example 1. The obtained peptide was subjected to analytical reversed phase high performance liquid chromatography [conditions were the same as above]; a single acute peak appeared at a retention time of 19.3 minutes. The molecular weight of the peptide was determined to be 3271 by FAB mass spectrometry (theoretical value = 3270.54).

Reference Examples 3 to 5

The partial peptide (1-13), the partial peptide (11-22) and the partial peptide (20-31) of the HTLV-I-associated peptide (1-31) of Reference Example 2 (SEQ ID NO:4) were obtained by solid phase synthesis and purification of peptide in the same manner as in Example 1, respectively. The obtained peptides were subjected to analytical reversed phase high performance liquid chromatography [conditions were the same

as above]; a single acute peak appeared at a retention time of 19.7 minutes, 18.2 minutes and 17.5 minutes, respectively. The molecular weight of the peptides were determined to be 1340, 1323 and 1311 by FAB mass spectrometry, respectively (theoretical value = 1340.33, 1323.34, 1310.45).

Reference Example 6

The HTLV-II-associated peptide represented by the formula:

Cys Pro Thr Thr Thr Pro Pro Pro Pro Pro Pro Pro Ser Pro

Glu Ala His Val Pro Pro Pro Tyr Val Glu Pro Thr Thr Thr

Gln Cys Phe (SEQ ID NO:5),

was obtained by solid phase synthesis and purification of peptide in the same manner as in Example 1. The obtained peptide was subjected to analytical reversed phase high performance liquid chromatography [conditions were the same as above]; a single acute peak appeared at a retention time of 18.5 minutes. The molecular weight of the peptide was determined to be 3264 by FAB mass spectrometry (theoretical value = 3263.54).

Reference Examples 7 to 8

The partial peptide (1-12) and the partial peptide (20-31) of the HTLV-II-associated peptide (1-31) of Reference Example 6 (SEQ ID NO:5) were obtained by solid phase synthesis and purification of peptide in the same manner as in Example 1, respectively. The obtained peptides were subjected to analytical reversed phase high performance liquid chromatography [conditions were the same as above]; a single acute peak appeared at a retention time of 17.4 minutes and 17.9 minutes, respectively. The molecular weight of the peptides were determined to be 1201 and 1363 by FAB mass spectrometry, respectively (theoretical value = 1201.34, 1363.46).

Example 3

Subject samples

| | |
|---|---|
| Sera from HTLV-I carriers: | 15 specimens |
| Sera from HTLV-II carriers: | 9 specimens |
| Normal human sera: | 96 specimens |

Determination by ELISA

Each serum specimen was examined for anti-HTLV-I antibody and anti-HTLV-II antibody by determining the absorbance by the following ELISA.

Each of the peptides obtained in Examples 1 and 2 or the peptides obtained in Reference Examples 1 to 8 was dissolved in 0.01 M carbonate buffer (pH 9.5). Each obtained peptide solution was added to a polystyrene enzyme immunoassay cup (Dynatech Laboratories Incorporation) at 100µl per cup and kept standing at 4°C for one night for peptide coating. After removing the peptide solution from each assay cup, 200 µl of 0.01 M phosphate buffered saline (hereinafter abbreviated PBS) containing 20% by volume of normal goat serum (NGS) was added, and each cup was kept standing at room temperature for 3 hours to block the nonspecific protein binding site. Then, after removing the PBS/NGS each assay cup was dried under reduced pressure.

To each assay cup described above, 100 µl of PBS containing 10% by volume of normal goat serum, as the serum diluent, was added, whereafter each subject serum (Sera from HTLV-I carriers:15 specimens, Sera from HTLV-II carriers:9 specimens, Normal human sera:96 specimens) was added so that the ratio of the serum diluent to the subject serum became 20 to 1 (by volume). After incubation at 37°C for 1 hour, each cup was washed with three portions of PBS containing 0.05% by volume of Tween 20.

To each assay cup thus treated, 100µl of a goat antihuman IgG antibody-peroxidase conjugate (diluted to an optimum concentration with PBS containing 10% by volume of normal goat serum) was added. After incubation at 37°C for 30 minutes, each cup was washed with three portions of PBS containing 0.05% by volume of Tween 20. Subsequently, to each assay cup thus treated, 100µl of coloring agent (prepared by dissolving o-phenylenediamine in a 0.1 M citrate-phosphate buffer (pH 5.6), containing 0.02% by volume of hydrogen peroxide, to a final concentration of 0.3% by weight) was added. After this mixture was kept standing at room temperature for 15 minutes, 100µl of 2 N sulfuric acid was added to stop the reaction, and the absorbance at 492 nm ($OD_{492}$) of the reaction mixture was determined.

Results

The assay results are shown in Table 2. A cut-off level was set from the $OD_{492}$ values of 96 normal human serum samples, and the subject serum was judged for the presence or absence of antibody against each peptide. The cut-off level was determined using the following equation:
mean of $OD_{492}$ value of normal human serum + 2SD(standard deviation)
According to the $OD_{492}$ value of each subject serum judged to be positive from the cut-off level obtained using the above equation, the subject sera were classified by antibody titer as follows: + for < 0.25, 2+ for 0.25-0.5, 3+ for 0.5-0.75, 4+ for 0.75-1.0, 5+ for > 1.0. As a result, in the ELISA using the HTLV-II-associated peptide obtained in Example 1, all subject sera from HTLV-II carriers reacted with the peptide and were judged to be positive (9/9), while none of the sera from HTLV-I carriers did (0/15). On the other hand, in the ELISA using the HTLV-I-associated peptide obtained in Reference Example 2, all subject sera from HTLV-I carriers reacted strongly and were judged to be positive (15/15), while the sera from HTLV-II carriers showed no such strong reaction (1/9). In other words, the ELISA using the peptide obtained in Example 1 specifically detected anti-HTLV-II antibody alone with high sensitivity, and thus proved to permit serologic discrimination between HTLV-I infection and HTLV-II infection. The ELISA using the HTLV-II-associated peptide obtained in Example 2 also proved that the sera from HTLV-II carriers reacts alone.

These findings demonstrate that ELISA using a peptide of the present invention makes it possible to eliminate the cross-reaction between HTLV-I and HTLV-II which could not be eliminated by the PA method or the ELISA using whole viral lysates as antigens.

To summarize, the use of a peptide of the present invention clearly discriminates between HTLV-I infection and HTLV-II infection which have been impossible to discriminate.

Table 2

| | HTLV-II-associated | | | | | HTLV-I-associated | | | | |
|---|---|---|---|---|---|---|---|---|---|---|
| Synthesis of peptide | Example | | Reference Example | | | Reference Example | | | | |
| | 1 | 2 | 6 | 7 | 8 | 1 | 2 | 3 | 4 | 5 |
| SEQ ID NO: | 1 | 2 | 5 | 5 (1-12) | 5 (20-31) | 3 | 4 | 4 (1-13) | 4 (11-22) | 4 (20-31) |
| Subject serum | Serum from HTLV-I carrier | | | | | | | | | |
| No. 1 | - | - | 5+ | - | 2+ | 5+ | 5+ | + | + | 3+ |
| 2 | - | - | 3+ | - | 2+ | 5+ | 5+ | - | + | 4+ |
| 3 | - | - | + | - | + | 5+ | 5+ | - | 2+ | 3+ |
| 4 | - | - | 5+ | - | + | 5+ | 5+ | - | 2+ | 5+ |
| 5 | - | - | 2+ | - | 2+ | 5+ | 5+ | - | 2+ | 5+ |
| 6 | - | - | - | - | + | 5+ | 5+ | - | 2+ | 3+ |
| 7 | - | - | + | - | + | 4+ | 5+ | - | 2+ | + |
| 8 | - | - | 2+ | - | - | 5+ | 5+ | - | 2+ | 4+ |
| 9 | - | - | 4+ | - | - | 5+ | 5+ | - | 3+ | + |
| 10 | - | - | - | - | - | 5+ | 5+ | - | 3+ | 5+ |
| 11 | - | - | + | - | - | 5+ | 5+ | - | 2+ | 4+ |
| 12 | - | - | + | - | - | 3+ | 5+ | - | 2+ | 3+ |
| 13 | - | - | 5+ | - | 2+ | 4+ | 5+ | 2+ | 3+ | 4+ |
| 14 | - | - | - | - | - | 5+ | 4+ | - | - | + |
| 15 | - | - | 3+ | - | - | 5+ | 5+ | - | 2+ | 3+ |
| Subject serum | Serum from HTLV-II carrier | | | | | | | | | |
| No.16 | 3+ | 2+ | 3+ | - | - | - | - | ND | ND | ND |
| 17 | 5+ | - | 2+ | - | 2+ | - | - | ND | ND | ND |
| 18 | 5+ | - | 3+ | - | - | - | + | - | - | - |
| 19 | 5+ | 3+ | 3+ | - | - | - | - | - | - | - |
| 20 | 3+ | - | - | - | + | - | - | - | - | - |
| 21 | 5+ | - | - | - | - | - | - | - | - | - |
| 22 | 5+ | 4+ | 5+ | - | - | - | - | - | - | - |
| 23 | 5+ | 4+ | 4+ | - | - | - | - | - | - | - |
| 24 | 2+ | - | - | - | - | - | - | - | - | - |
| Cut-off level | .175 | .195 | .236 | .212 | .170 | .091 | 191 | .365 | .209 | .575 |

ND: no determination

SEQUENCE LISTING

SEQ ID NO : 1

LENGTH: 26

TYPE: amino acid

TOPOLOGY: linear

MOLECULE TYPE: peptide

SEQUENCE

Phe Ile Thr Ser Glu Pro Thr Gln Pro Pro Pro Thr Ser Pro Pro Leu
1 5 10 15

Val His Asp Ser Asp Leu Glu His Val Leu
20 25

SEQ ID NO : 2

LENGTH: 12

TYPE: amino acid

TOPOLOGY: linear

MOLECULE TYPE: peptide

SEQUENCE

Pro Pro Ser Pro Glu Ala His Val Pro Pro Pro Tyr
1 5 10

SEQ ID NO : 3

LENGTH: 25

TYPE: amino acid

TOPOLOGY: linear

MOLECULE TYPE: peptide

SEQUENCE

Phe Leu Asn Thr Glu Pro Ser Gln Leu Pro Pro Thr Ala Pro Pro Leu
 1 5 10 15
Leu Pro His Ser Asn Leu Asp His Ile
 20 25

SEQ ID NO : 4

LENGTH: 31

TYPE: amino acid

TOPOLOGY: linear

MOLECULE TYPE: peptide

SEQUENCE

Pro Pro Pro Pro Ser Ser Pro Thr His Asp Pro Pro Asp Ser Asp Pro
 1 5 10 15
Gln Ile Pro Pro Pro Tyr Val Glu Pro Thr Ala Pro Gln Val Leu
 20 25 30

SEQ ID NO : 5

LENGTH: 31

TYPE: amino acid

TOPOLOGY: linear

MOLECULE TYPE: peptide

SEQUENCE

Cys Pro Thr Thr Thr Pro Pro Pro Pro Pro Pro Pro Ser Pro Glu Ala
 1 5 10 15
His Val Pro Pro Pro Tyr Val Glu Pro Thr Thr Thr Gln Cys Phe
 20 25 30

## Claims

1. A peptide comprising a HTLV-II-derived peptide fragment capable of specifically binding to anti-HTLV-II antibodies, which does not react with anti-HTLV-I antibodies.

2. The peptide according to claim 1 of the formula:

Phe Ile Thr Ser Glu Pro Thr Gln Pro Pro Pro Thr Ser Pro Pro Leu Val His Asp Ser Asp Leu Glu His Val Leu (SEQ ID NO:1).

**3.** The peptide according to claim 1 of the formula:

Pro Pro Ser Pro Glu Ala His Val Pro Pro Pro Tyr (SEQ ID NO:2).

**4.** An assay reagent for the detection of anti-HTLV-II antibodies, comprising at least one peptide of any one of claims 1 to 3.

**5.** A method for the detection of anti-HTLV-II antibodies using an assay reagent of claim 4.

**6.** The method according to claim 5, wherein the detection of anti-HTLV-II antibodies is carried out by an enzyme immunoassay.

**7.** A kit comprising at least one peptide of any one of claims 1 to 3.